# EUROPÄISCHE PATENTANMELDUNG

(11) **EP 1 216 651 A1**
(43) Veröffentlichungstag der Anmeldung: **26.06.2002**
(21) Anmeldenummer: 00127951.2
(22) Anmeldetag: 21.12.2000
(51) Int. Cl.: A61B 5/00, A61B 17/00

(54) **Kabelloses medizinisches Erfassungs- und Behandlungssystem**

(71) Anmelder: BrainLAB AG, 85551 Kirchheim/Heimstetten (DE)
(72) Erfinder: Birkenbach, Rainer, 85586 Poing (DE); Müller, Günter, 85570 Markt Schwaben (DE)
(74) Vertreter: Schwabe - Sandmair - Marx

(57) **Zusammenfassung**

Die Erfindung betrifft ein System für medizinische Erfassungen und Behandlungen mit mehreren Behandlungs- und/oder Erfassungsgeräten die im Rahmen einer medizinischen Behandlung, insbesondere einer chirurgischen oder strahlentherapeutischen bzw. radiochirurgischen Operation, Daten untereinander austauschen, bei dem der Datenaustausch über Funkschnittstellen in bzw. an den Geräten erfolgt.

## Beschreibung

Die vorliegende Erfindung betrifft ein System für medizinische Erfassungen und Behandlungen mit mehreren Behandlungs- und/oder Erfassungsgeräten, die im Rahmen einer medizinischen Behandlung, insbesondere einer chirurgischen oder strahlentherapeutischen bzw. radiochirurgischen Operation, Daten untereinander austauschen.

Im Zuge der technischen Weiterentwicklung auch im medizinischen Bereich wird es immer wichtiger, dass medizinische Behandlungs- und/oder Erfassungsgeräte untereinander Daten austauschen. So arbeiten heute schon mehrere Geräte computergesteuert mittels Datenaustausch beispielsweise in einem Operationsraum zusammen, wenn die Operation durch ein medizinisches Navigations- oder Trackingsystem unterstützt wird. Hierbei werden zum Beispiel Lagedaten oder Daten über den derzeitigen Zustand des Patienten erfasst (zum Beispiel durch Kameras oder interoperative bildgebende Verfahren wie zum Beispiel intraoperative Kernspintomographen bzw. Computertomographen) und diese Daten werden an eine zentrale Rechner- bzw. Steuerungseinheit übergeben, welche nach einer computergestützten Verarbeitung auf einem Bildschirm behandlungsunterstützende bzw. behandlungsleitende Informationen für den behandelnden Arzt ausgibt. Da es des Öfteren vorkommt, dass eine Vielzahl solcher Behandlungsgeräte eingesetzt wird, und diese Geräte gemäß dem Stand der Technik ihre Daten über Kabelverbindungen austauschen, kommt es meist in Operationssälen zu einer Anhäufung verlegter Kabel, die behandelnde Ärzte und Hilfspersonal stören oder in ihrer Bewegungsfreiheit einschränken. Die Verkabelungen erfordern, dass einige Geräte sehr nah bei der Patientenliege aufgestellt werden, wodurch eine weitere Einschränkung der Bewegungsfreiheit erfolgt. Die Situation wird noch dadurch verschlimmert, dass diese Geräte grundsätzlich eine Stromversorgung benötigen und aus diesem Grund noch mehr Kabel verlegt werden müssen. Außerdem ist der Geräteaufbau im Operationssaal durch die Notwendigkeit der Verlegung einer großen Anzahl von Kabeln sehr aufwändig, hierfür muss technisches Hilfspersonal des Öfteren über längere Zeit vorbereitend arbeiten. Fehlerquellen entstehen, falls versehentlich Anschlüsse beschädigt werden oder korrodieren oder anderweitig Kontaktprobleme auftreten, und einzelne Behandlungsgeräte, zum Beispiel Ultraschallsonden, können schwerlich steril verpackt werden, da sie an der Kabelverbindung hängen.

Es ist deshalb die Aufgabe der vorliegenden Erfindung, ein System für medizinische Erfassungen und Behandlungen mit mehreren Behandlungs- und/oder Erfassungsgeräten bereitzustellen, welches die obigen Nachteile des Standes der Technik überwindet. Insbesondere soll das System die Verbindung der Geräte zum Datenabtausch optimieren. Auch soll eine sichere Datenübertragung gewährleistet werden, und speziell soll auch die Bewegungsfreiheit des Behandlungsteams verbessert werden.

Diese Aufgabe wird erfindungsgemäß gelöst durch ein System für medizinische Erfassungen und Behandlungen mit mehreren Behandlungs- und/oder Erfassungsgeräten, die im Rahmen einer medizinischen Behandlung, insbesondere einer chirurgischen oder strahlentherapeutischen bzw. radiochirurgischen Operation, Daten untereinander austauschen bei dem der Datenaustausch über Funkschnittstellen in bzw. an den Geräten erfolgt. Mit anderen Worten wird hinsichtlich des Datenaustausches ein kabelloses System bereitgestellt, was den großen Vorteil hat, dass die Kabel, die früher zum Datenaustausch benötigt wurden, nunmehr im Operationssaal nicht mehr vorhanden sind und damit auch nicht stören können. Da solche Systeme grundsätzlich die in einem Operationssaal entstehenden Abstände ohne weiteres überwinden können, besteht ferner vorteilhafterweise die Möglichkeit, alle Geräte, die nicht unbedingt in der Nähe der Patientenliege vorhanden sein müssen, in einer Entfernung hiervon aufzustellen, so dass sie dem Behandlungsteam nicht mehr im Weg stehen. Behandlungsapparaturen bzw. behandlungsunterstützende Apparaturen können ohne weiteres und ohne Rücksicht auf Kabellängen an den am besten geeigneten Orten aufgestellt und sogar von der Decke abgehängt werden. Außerdem wird die Handhabung von Handgeräten oder Sonden nicht mehr durch störende Datenübertragungskabel erschwert.

Die beiliegenden Unteransprüche definieren bevorzugte Ausführungsformen der vorliegenden Erfindung.

So kann die Funkübertragung innerhalb eines Behandlungsraumes über kurze Distanzen in einem ISM (Industrial, Scientific, Medical) -Band erfolgen, und zwar bei niedriger Energie, insbesondere bei ungefähr 1 mW, im Hochfrequenzbereich, insbesondere in einem Bereich um 2,4 GHz (ISM Band), insbesondere bei 2,4 GHz bis 2,4835 GHz (Blue Tooth-Band), und alternativ im 900 MHz ISM Band und/oder im ISM Band von 5,150 bis 5,875 GHz. Für solche Funkübertragungen stehen also unter anderem internationale Standards (Blue Tooth-Standard; HyperLAN) zur Verfügung, die sich speziell auch für die Verwendung mit der vorliegenden Erfindung eignen.

Eines oder mehrere der folgenden Geräte können im Rahmen der vorliegenden Erfindung mit Funkschnittstellen zur Kommunikation ausgestattet sein:
- Kamerasystem, insbesondere Infrarotkamerasystem, zum Beispiel zur Verwendung mit einem medizinischen Navigations- und Trackingsystem;
- Audioeinrichtung, zum Beispiel zur Verwendung mit einem medizinischen Navigations- und Trackingsystem;
- Videoeinrichtung, insbesondere Bildschirm und Videosignal-Erzeugungsvorrichtungen, auch Head-Mounted-Displays, zum Beispiel zur Verwendung mit einem medizinischen Navigations- und Trackingsystem;
- Mikroskopeinrichtung, zum Beispiel zur Verwendung mit einem medizinischen Navigations- und Trackingsystem;
- Ultraschall-Erfassungseinrichtung und/oder Einrichtung zum Verarbeiten von erfassten Ultraschallsignalen, zum Beispiel zur Verwendung mit einem medizinischen Navigations- und Trackingsystem;
- Roboter, insbesondere medizinischer Roboter, zum Beispiel zur Verwendung mit einem medizinischen Navigations- und Trackingsystem;
- Patientenlagerungen, insbesondere Patientenliegen, zum Beispiel zur Verwendung mit einem medizinischen Navigations- und Trackingsystem;
- Bilderzeugungsgeräte, insbesondere medizinische, bevorzugt zur Erfassung von Volumendatensätzen, wie Tomographen, zum Beispiel zur Verwendung, auch intraoperativen Verwendung mit einem medizinischen Navigations- und Trackingsystem;
- AGV-Geräte (Automatic Guided Vehicles; Fahrerlose Transportsysteme);
- Behandlungsapparaturen und Behandlungsinstrumente, insbesondere medizinische bzw. chirurgische Instrumente, zum Beispiel zur Verwendung mit einem medizinischen Navigations- und Trackingsystem;
- Endoskope;
- C-Bogen-Röntgengeräte bzw. Fluoroskopiegeräte.

Hervorzuheben ist für die oben genannten Geräte die Möglichkeit der optimalen Platzierung bzw. Anordnung im Behandlungs- bzw. Operationsraum, und es entstehen auch andere Vorteile. Beispielsweise können Behandlungsinstrumente, wie zum Beispiel Sonden, als "intelligente" Sonden ausgestaltet werden, die über ihre Schnittstelle über eine Betätigungseinrichtung (zum Beispiel eine Art Mouseklick) Daten über ihren momentanen Ort oder Zustand oder den Ort oder Zustand der erfassten Strukturen weitergeben. Was die Verwendung mit Endoskopen betrifft, so kann bei der Verwendung eines Miniatursenders an der Endoskopsonde die Verkabelung, die normalerweise aus einer Stromzufuhr, einer Lichtversorgung und einem Strang für die Übersendung von Videosignalen besteht, zumindest um den letztgenannten Videosignal-Strang reduziert werden, wobei grundsätzlich die Möglichkeit besteht, die Endoskopsonden und ihren Kabelanhang zu verkleinern und damit auch leichter und weniger invasiv in bisher nicht zugängliche Zielvolumina einzudringen.

Die Geräte können vorzugsweise Daten zu ihrer eigenen Identifizierung und/oder erfasste medizinische Daten und/oder eigene oder fremde Lagedaten übertragen. Insbesondere die Übertragung von Daten zur Identifizierung der Geräte macht das System gegenüber dem Stand der Technik sehr sicher, da Fehler, wie sie durch fehlerhafte Kabelverbindungen entstehen, hierdurch ausgeschlossen werden. Es ist möglich, die Funkübertragung für ein Gerät auf mehreren, wechselnden Kanälen durchzuführen, bis ein Kanal gefunden ist, der die Datenübertragung mit ausreichender Genauigkeit erlaubt, wodurch die Sicherheit der Datenübertragung insgesamt verbessert wird. Hierbei suchen Sender und Empfänger so lange nach einem geeignetem Kanal, bis die optimalen Voraussetzungen für die Datenübertragung geschaffen sind. Es kann eine Kodierungsvariation erfolgen, welche sicherstellt, dass die Zuordnung der Geräte in richtiger Weise erfolgt. Eine Kennung für jeden Sender stellt schon am Anfang sicher, dass die Kommunikationssignale von Anfang an den richtigen Geräten zugeordnet werden.

Bei einer vorteilhaften Ausgestaltung des erfindungsgemäßen Systems weist dieses eine zentrale, computergestützte Empfangs-, Sende- und Auswertungseinheit auf, welche auf der Basis der ausgetauschten Daten Erkennungs-, Regelungs- und Steuerungsabläufe ausführt bzw. ausgewertete Daten weitergibt. Hierzu eignet sich ein Computer, wie er beispielsweise schon heute im Rahmen von computergestützten Navigations- bzw. Trackingsystemen eingesetzt wird, wobei dieser Computer vorteilhafterweise nicht mehr direkt an der Patientenliege aufgestellt werden muss, sondern abseits im Operationssaal stehen kann. Insgesamt eignet sich das erfindungsgemäße kabellose, medizinische Erfassungs- und Behandlungssystem besonders zur Integration in ein medizinisches Navigations- und Trackingsystem mit den zugehörigen Behandlungseinrichtungen.

Ein weiterer großer Vorteil hinsichtlich der Bewegungsfreiheit und der Verringerung der Anzahl der auszulegenden bzw. vorzusehenden Kabelverbindungen entsteht, wenn die Geräte, wie dies gemäß einer bevorzugten Ausführungsform der vorliegenden Erfindung vorgesehen ist, akku- bzw. batteriebetrieben sind. Da einige der bei solchen Behandlungen eingesetzten Geräte eine Stromversorgung benötigen, sind für ihren Betrieb herkömmlicherweise noch immer Stromversorgungskabel notwendig, welche den schon beschriebenen störenden Einfluss haben. Im Rahmen des Fortschrittes bei der Batterie- bzw. Akkutechnologie ist es grundsätzlich im Rahmen der Erfindung möglich, auch diese Stromverbindungen zu eliminieren und Geräte bereitzustellen, die den Datenabtausch kabellos erledigen und ihre eigene Stromversorgung verwenden. Mit dieser Maßnahme kann nun im Wesentlichen ein kabelloser Operationssaal bereitgestellt werden. Durch den Wegfall von Kabeln können die Desinfektionsmaßnahmen im Operationssaal weitgehend vereinfacht werden und elektrische Schnittstellen mit anfälligen Steckkontakten, die ebenfalls schlecht oder gar nicht zu reinigen sind, entfallen. Technisch erhöht sich dadurch die Betriebssicherheit bei gleichzeitigen Kosteneinsparungen, da beispielsweise aufwändige Isolationsmaßnahmen mit Optokopplern, Übertragern usw. sowie ESD-Schutzschaltungen entfallen können.

Die Geräte, die kabellos Daten austauschen, können in einem einzigen Behandlungs- bzw. Operationssaal untergebracht sein. Es besteht jedoch auch die Möglichkeit, eine Funkverbindungseinrichtung zwischen mehreren Räumen herzustellen und so auch Geräte kabellos in Datenaustausch zu bringen, die in verschiedenen Räumen untergebracht sind. So kann zum Beispiel ein in einem separaten Raum aufgestellter Kernspintomograph immer mit aktuellen Daten über den Patientenzustand, insbesondere die aktuelle Patientenanatomie versorgt werden und unmittelbar unter den richtigen Voraussetzungen zum Einsatz gebracht werden, obwohl der Patient zur Tomographieerfassung während der Operation oder nach der Operation zur Kontrolle in einen solchen anderen Raum gebracht werden muss.

Die Übertragung von Video- bzw. Bildsignalen könnte sich wegen der großen Menge der zu übertragenden Daten eventuell schwierig gestalten. Die vorliegende Erfindung bietet hierzu mehrere Lösungen an. Beispielsweise kann eine Einrichtung zum Komprimieren der Videobzw. Bildsignaldaten zwischengeschaltet werden. Ebenfalls besteht die Möglichkeit, eine Einrichtung zur Umwandlung digitaler Video- bzw. Bildsignale in Datensignale mit geringem Umfang, zum Beispiel Composite-Signale bereitzustellen, und weiterhin ist es im Rahmen der vorliegenden Erfindung möglich, die Video- bzw. Bildsignale mit einer geeigneten Einrichtung aufzuspalten und parallel anteilsweise zu übertragen, insbesondere mit Mehrkanal-RF-Transmissions-Verbindungen.

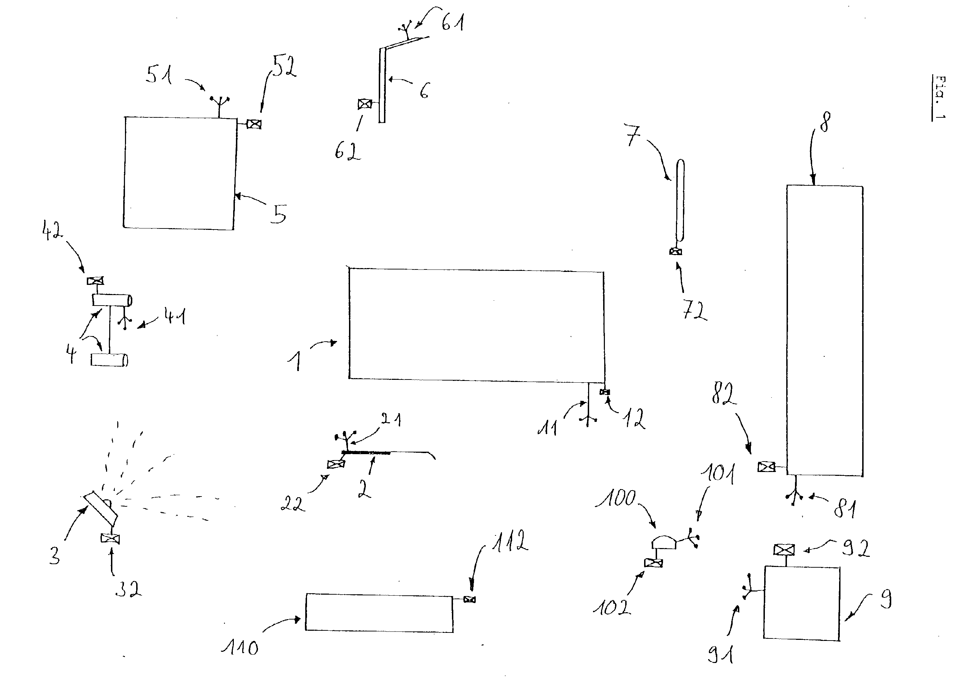

Die Erfindung wird im Weiteren anhand eines Ausführungsbeispiels mit Hilfe der beiliegenden Zeichnung näher erläutert. Die Zeichnung zeigt einen schematischen Überblick über einen Operationssaal mit darin aufgestellten Behandlungs- bzw. Erfassungsgeräten. In dem Operationssaal vorgesehen ist eine Patientenliege 1, eine Sonde 2, die hier ganz allgemein für Behandlungsinstrumente wie Sonden, Pinzetten, Skalpelle, usw. steht, ein Infrarotlichtstrahler 3, ein Infrarot-Kamerasystem 4, ein chirurgisches Mikroskop 5, ein chirurgischer Roboter 6, ein Bildschirm 7 als Video-Behandlungsunterstützung, ein intraoperativer Kernspintomograph 8, der verfahrbar so angeordnet ist, dass er über die Patientenliege 1 gefahren werden kann, ein Ultraschallgerät 9 und eine dazugehörige Ultraschallsonde 100, sowie eine zentrale Computereinheit 110. Die letztgenannte Computereinheit 110 steuert, regelt und verarbeitet die Vorgänge und Informationen im System, wobei es durchaus auch möglich ist, dass einzelne der vorher genannten Geräte selbst schon Steuerungs- und Verarbeitungseinheiten für Informationen aufweisen.

Alle dargestellten Geräte sind gemäß der vorliegenden Erfindung mit Funkschnittstellen ausgestattet, die schematisch durch ein Kästchen dargestellt sind, das jeweils bezeichnet ist mit dem Bezugszeichen des Gerätes und einer dahinterstehenden 2. So hat beispielsweise die Patientenliege 1 eine Funkschnittstelle mit dem Bezugszeichen 12, die Sonde 2 hat eine Funkschnittstelle mit dem Bezugszeichen 22 usw.. Bis auf den Computer 110 und den Infrarotstrahler 3 sind auch alle dargestellten Geräte bzw. Geräteeinheiten mit Referenzierungsmarkern versehen, welche es dem aus diesen Referenzierungsmarkern, dem Kamerasystem 4, dem Infrarotstrahler 3 und dem Computer 110 bestehenden Navigations- und Trackingsystem gestatten, die räumliche Lage der Geräte im Operationsraum zu erfassen und zu verfolgen und damit eine navigations- und bildunterstützte Behandlung zu ermöglichen. Die Referenzierungsmarker bzw. Referenzierungsmarkeranordnungen (schematisch sind jeweils Adapter mit je drei Markern an jedem Gerät dargestellt) sind mit dem Bezugszeichen des jeweiligen Gerätes und einer dahinterstehenden 1 versehen, so trägt beispielsweise die Patientenliege 1 die Markeranordnung 11, die Sonde 2 trägt die Markeranordnung 21 usw..

Die Navigation, die in diesem Falle auf infrarotreflektierenden Markeranordnungen und einem entsprechenden Kamerasystem 4 mit Infrarotabstrahlung 3 beruht, ist grundsätzlich bekannt und wird hier nur kurz erläutert. Die Markeranordnungen an den jeweiligen Geräten reflektieren das Licht der Infrarotlichtquelle 3, und die entsprechenden von den Kameras des Kamerasystems 4 empfangenen Reflektionssignale werden ausgewertet, so dass die Raumlage eines jeden mit einer Markeranordnung versehenden Gerätes bestimmt werden kann. Der Computer koordiniert diese Informationen und verwendet sie, um auf dem Schirm 7 eine Bildunterstützung auszugeben. Schon allein zu diesem Zweck war es früher zumindest notwendig, dass vom Kamerasystem 4 und vom Bildschirm 7 Kabelverbindungen zum Computer 110 bestanden bzw. das Kamerasystem 4, der Computer 110 und der Bildschirm 7 zusammen mit der Infrarotlichtquelle 3 an einem einzigen Gestell befestigt waren, und zwar in einer Weise, die eine eindeutige Lagebestimmung dieser Teile erlaubte.

Schon für dieses Teilsystem bringt die vorliegende Erfindung, bei welcher der Datenaustausch nunmehr über die Funkschnittstellen 112, 32, 42 und 72 erfolgt, erhebliche Vorteile mit sich, da die Einzelkomponenten nicht mehr zusammen an einem Gestell in der Nähe der Patientenliege 1 vorgesehen werden müssen, sondern grundsätzlich die Möglichkeit besteht, die Komponenten aus dem Bereich um die Patientenliege herauszuverlagern und zwar möglichst an besonders geeigneten Stellen. So kann beispielsweise das Kamerasystem 4 zusammen mit dem Infrarotlichtabstrahler 3 an der Decke des Operationsraumes befestigt werden, der Computer 110 kann ohne weiteres an die Wand des Behandlungsraumes geschoben werden, und der Bildschirm 7 kann beispielsweise durch eine Halterung von der Decke aus, gerade da angebracht werden, wo ihn der behandelnde Arzt besonders gut einsehen kann. Auch die Verwendung größerer Wandbildschirme oder Projektoren ist grundsätzlich möglich. Die vorgenannten Komponenten des Navigations- bzw. Trackingsystems können deshalb ohne weiteres so angebracht werden, dass sie den Arzt bzw. das Behandlungsteam in keiner Weise stören und darüber hinaus noch ihrer Funktion gemäß einen optimalen Platz einnehmen.

Obiges gilt grundsätzlich für alle in einem Operationsraum anzuordnenden Geräte und Instrumente, und auf der Zeichnung sind zusätzlich noch die oben genannten Einheiten dargestellt, die im Rahmen einer Operation Verwendung finden könnten. So trägt das chirurgische Mikroskop 5 ebenfalls eine Schnittstelle 52. Es kann weit ab von der Patientenliege verbleiben, solange es nicht gebraucht wird und in einfacher Weise kabellos dann an die Patientenliege gebracht werden, wenn es zum Einsatz kommen soll. Dabei ist seine Lage im Navigationssystem über die Markeranordnung 51 bestimmt und die vom Mikroskop 5 erfassten Daten können über die Schnittstelle 52 zum Computer 110 übertragen werden, der dann über seine Schnittstelle 112 die erforderlichen Bildsignale an den Bildschirm 7 und dessen Schnittstelle 72 funkt. Obiges gilt grundsätzlich auch für den Behandlungsroboter 6 und das intraoperative Kernspintomographiesystem 8, die beide im Navigations- bzw. Trackingsystem eingebunden sind und aufgrund aktueller Daten operieren bzw. solche aktuellen Daten erfassen.

Ein weiteres funkverbundenes Erfassungsgerät ist das Infrarotgerät 9 mit der Markeranordnung 91 und der Funkschnittstelle 92 sowie die zugehörige Infrarotsonde 100 mit der Markeranordnung 101 und der Schnittstelle 102. Durch die Funkschnittstelle 102 durch die die Sonde 100 Daten mit dem Infrarotgerät 9 austauschen kann, wird diese Sonde besonders gut handhabbar, da für den Datenaustausch kein Kabel mehr vorgesehen werden muss. Ebenso wie alle anderen dargestellten Geräte kann die Infrarotsonde akku- bzw. batteriebetrieben sein, wodurch der zusätzliche Vorteil entsteht, dass grundsätzlich keinerlei Kabel mehr benötigt werden. Im Falle der Ultraschallsonde 100 bedeutet dies, dass der Arzt diese Sonde völlig frei bewegen kann und sogar eine Zwischensterilisierung möglich ist. Es ist denkbar, alle dargestellten Geräte vor und nach der Verwendung an Dockingstations anzuschließen, welche die Akkumulatoren wieder aufladen, so dass bei Operationsbeginn jedes Geräte über lange Zeit einsetzbar ist, ohne eine Stromkabelversorgung zu benötigen. Auch sind Zwischenlösungen möglich, da eventuell festmontierte Geräte, wie zum Beispiel das Kamerasystem 4 und der Bildschirm 7 über Deckenleitungen mit Strom versorgt werden können, ohne dass Behinderungen für das Operationsteam entstehen.

Hinzuweisen ist schließlich noch auf die Möglichkeit, auch die Sonde 2 mit einer Funkschnittstelle zu versehen. Es würde dadurch eine Art "intelligente" Sonde entstehen, die zur Momentanpositionserfassung dienen kann. Die Raumposition der Sondenspitze ist über die Markeranordnung 21 im Navigationssystem jederzeit bekannt. Wenn ein Arzt nunmehr die Spitze auf einen Punkt des Patientenkörpers führt, der selbst nur schwer im Navigationssystem erfassbar ist, kann er - beispielsweise über einen Schalter (nicht dargestellt) an der Sonde 2 eine Art "Mouseklick" abgeben. Dieses Signal würde vom Computer 110 registriert und es könnte dann positiv festgestellt werden, dass der Körperpunkt des Patienten, auf dem die Sondenspitze ruht, sich im Moment des Mouseklicks an einer bestimmten Raumposition befindet. Dies ist insbesondere dann von Vorteil, wenn Körperstrukturen sich während einer Operation verändern, beispielsweise das Gehirn beim Öffnen der Schädeldecke oder bei der Entfernung einer Läsion.

Natürlich können durch die vorgenannte Methode auch künstliche Marker am Patienten neu referenziert werden.

Über die Funkschnittstellen können Daten zur Identifizierung der jeweiligen Geräte, erfasste medizinische Daten, eigene oder fremde Lagedaten oder Kombinationen aus diesen sowie andere relevante Daten übertragen werden.

## Patentansprüche

1. System für medizinische Erfassungen und Behandlungen mit mehreren Behandlungsund/oder Erfassungsgeräten, die im Rahmen einer medizinischen Behandlung, insbesondere einer chirurgischen oder strahlentherapeutischen bzw. radiochirurgischen Operation, Daten untereinander austauschen, **dadurch gekennzeichnet, dass** der Datenaustausch über Funkschnittstellen in bzw. an den Geräten erfolgt.

2. System nach Anspruch 1, **dadurch gekennzeichnet, dass** die Funkübertragung innerhalb eines Behandlungsraums über Kurzdistanzen in einem ISM (Industrial, Scientific, Medical)-Band erfolgt, und zwar bei niedriger Energie, insbesondere ungefähr 1 mW, im Hochfrequenzbereich.

3. System nach Anspruch 1 oder 2, **dadurch gekennzeichnet, dass** die Funkübertragung im Bereich um 2,4 GHz und insbesondere bei 2,4 bis 2,4835 GHz oder im Bereich um 5 GHz, speziell im Bereich von 5,150 bis 5,875 GHz stattfindet.

4. System nach Anspruch 1 oder 2, **dadurch gekennzeichnet, dass** die Funkübertragung im Bereich des 900 MHz ISM Bandes stattfindet.

5. System nach einem der Ansprüche 1 bis 4, **dadurch gekennzeichnet, dass** eines oder mehrere der folgenden Geräte mit Funkschnittstellen zur Kommunikation ausgestattet ist bzw. sind:
- Kamerasystem (4), insbesondere Infrarotkamerasystem, zum Beispiel zur Verwendung mit einem medizinischen Navigations- und Trackingsystem;
- Audioeinrichtung, zum Beispiel zur Verwendung mit einem medizinischen Navigations- und Trackingsystem;
- Videoeinrichtung, insbesondere Bildschirm (7) und Videosignal-Erzeugungsvorrichtungen, auch Head-Mounted-Displays, zum Beispiel zur Verwendung mit einem medizinischen Navigations- und Trackingsystem;
- Mikroskopeinrichtung, zum Beispiel zur Verwendung mit einem medizinischen Navigations- und Trackingsystem;
- Ultraschall-Erfassungseinrichtung (100) und/oder Einrichtung (9) zum Verarbeiten von erfassten Ultraschallsignalen, zum Beispiel zur Verwendung mit einem medizinischen Navigations- und Trackingsystem;
- Roboter, insbesondere medizinischer Roboter (6), zum Beispiel zur Verwendung mit einem medizinischen Navigations- und Trackingsystem;
- Patientenlagerungen, insbesondere Patientenliegen (1), zum Beispiel zur Verwendung mit einem medizinischen Navigations- und Trackingsystem;
- Bilderzeugungsgeräte, insbesondere medizinische, bevorzugt zur Erfassung von Volumendatensätzen, wie Tomographen (8), zum Beispiel zur Verwendung, auch intraoperativen Verwendung mit einem medizinischen Navigations- und Trackingsystem;
- AGV-Geräte (Automatic Guided Vehicles; Fahrerlose Transportsysteme);
- Behandlungsapparaturen und Behandlungsinstrumente, insbesondere medizinische bzw. chirurgische Instrumente (2), zum Beispiel zur Verwendung mit einem medizinischen Navigations- und Trackingsystem;
- Endoskope;
- C-Bogen-Röntgengeräte bzw. Fluoroskopiegeräte.

6. System nach einem der Ansprüche 1 bis 5, **dadurch gekennzeichnet, dass** die Geräte Daten zu ihrer Identifizierung und/oder erfasste medizinische Daten und/oder eigene oder fremde Lagedaten übertragen.

7. System nach einem der Ansprüche 1 bis 6, **dadurch gekennzeichnet, dass** es eine zentrale, computergestützte Empfangs-, Sende- und Auswertungseinheit (110) aufweist, welche auf der Basis der ausgetauschten Daten Erkennungs-, Regelungs- und Steuerungsabläufe ausführt bzw. ausgewertete Daten weitergibt.

8. System nach einem der Ansprüche 1 bis 7, **dadurch gekennzeichnet, dass** es integriert ist in ein medizinisches Navigations- bzw. Trackingsystem, mit den zugehörigen Behandlungseinrichtungen.

9. System nach einem der Ansprüche 1 bis 8, **dadurch gekennzeichnet, dass** die Geräte akku- bzw. batteriebetrieben sind.

10. System nach einem der Ansprüche 1 bis 9, **dadurch gekennzeichnet, dass** die Geräte in einem Behandlungs- bzw. Operationsraum untergebracht sind.

11. System nach einem der Ansprüche 1 bis 9, **dadurch gekennzeichnet, dass** die Geräte in mehreren Räumen untergebracht sind und eine Funkverbindungseinrichtung zwischen den Räumen bereitgestellt wird.

12. System nach einem der Ansprüche 1 bis 11, **dadurch gekennzeichnet, dass** zur Übertragung von Video- bzw. Bildsignalen eine oder mehrere der folgenden Einrichtungen bereitgestellt wird bzw. werden:
- eine Einrichtung zur Komprimierung der Video- bzw. Bildsignaldaten;
- eine Einrichtung zur Umwandlung der Video- bzw. Bildsignaldaten in Datensignale mit geringem Umfang, zum Beispiel Composite-Signale;
- eine Einrichtung zum Aufspalten und zur parallelen Übertragung von Anteilen der Video- bzw. Bildsignaldaten, insbesondere mit Mehrkanal-RF-Transmissions-Verbindungen.
